(19) Europäisches Patentamt / European Patent Office / Office européen des brevets

(11) **EP 3 111 351 B1**

(12) **EUROPEAN PATENT SPECIFICATION**

(45) Date of publication and mention
of the grant of the patent:
**20.12.2023 Bulletin 2023/51**

(21) Application number: **15755569.9**

(22) Date of filing: **27.02.2015**

(51) International Patent Classification (IPC):
**A61B 5/08** *(2006.01)*    **A61B 5/11** *(2006.01)*
**A61B 5/00** *(2006.01)*    **G16H 10/60** *(2018.01)*
**G16H 40/63** *(2018.01)*    **G16H 50/20** *(2018.01)*
**G16H 50/30** *(2018.01)*    **A61B 5/113** *(2006.01)*

(52) Cooperative Patent Classification (CPC):
**G16H 50/30; A61B 5/0816; A61B 5/1118;
A61B 5/113; A61B 5/4866; A61B 5/6823;
A61B 5/7253; A61B 5/7264; A61B 5/7275;
A61B 5/7282; G16H 10/60; G16H 40/63;
G16H 50/20;** A61B 5/0022; A61B 5/0024;    (Cont.)

(86) International application number:
**PCT/IB2015/051456**

(87) International publication number:
**WO 2015/128842 (03.09.2015 Gazette 2015/35)**

(54) **DEVICE AND METHOD FOR MONITORING RESPIRATORY EFFORT USING ENERGY INDEX**

VORRICHTUNG UND VERFAHREN ZUR ÜBERWACHUNG DER ATEMANSTRENGUNG MITHILFE EINES ENERGIEINDEX

DISPOSITIF ET PROCÉDÉ DE SURVEILLANCE D'EFFORT RESPIRATOIRE À L'AIDE D'UN INDICE D'ÉNERGIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2014 US 201461945298 P**

(43) Date of publication of application:
**04.01.2017 Bulletin 2017/01**

(73) Proprietors:
• **Technion Research & Development Foundation Ltd.**
**3200000 Haifa (IL)**
• **Rambam Health Corporation**
**3525408 Haifa (IL)**

(72) Inventors:
• **LANDESBERG, Amir**
**3460321 Haifa (IL)**
• **ZIBOLI, Yaakov**
**4424505 Kfar Saba (IL)**
• **RISPLER, Shmuel**
**3271443 Haifa (IL)**

(74) Representative: **Kirkpatrick**
**Avenue Wolfers, 32**
**1310 La Hulpe (BE)**

(56) References cited:
EP-A1- 2 289 404    WO-A1-2005/018737
WO-A1-2009/124297    WO-A1-2013/177621
US-A1- 2004 073 128    US-A1- 2007 238 938
US-A1- 2010 204 550    US-A1- 2013 072 806
US-A1- 2013 116 578    US-B1- 6 527 729
US-B1- 6 581 595

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
A61B 2505/07; A61B 2562/0219

**Description**

FIELD OF THE INVENTION

The present invention is related to a method and a device for monitoring a breathing energy index (EEI). The method is particularly useful, without limitation, for monitoring patients at home, for optimization of treatment of patients with heart failure, for determining the effectiveness of the treatment during electively hospitalization for stabilization of deteriorating cardiac decompensation, and for decision making before discharging patients from the hospital.

BACKGROUND OF THE INVENTION

Heart failure (HF) is a clinical syndrome resulting from structural or functional cardiac disorders that impair the ability of the heart to eject blood adequate for the needs of the body. The main manifestations in this syndrome are subjective symptoms of dyspnea (due to pulmonary congestion) and fatigue. These subjective symptoms are associated with various measureable objective signs. The objective signs include: fluid retention (weight gain), decreased exercise tolerance (maximal walk distance), decrease in the total oxygen consumption, and changes in the hemodynamic indices (as decrease in the cardiac output, increase in the left atrial pressure, increase in the end-diastolic pulmonary pressure). Heart failure is associated with changes in cardiac ejection fraction (systolic heart failure) and\or impairment in the cardiac filling (diastolic heart failure) that are measured by various imaging techniques. A prominent sign of heart failure is the development of lung congestion that is most commonly assessed by monitoring the thoracic impedance. Although there are various objective signs for heart failure, the quality of life is quantified by subjective symptoms, and the New-York Heart association (NYHA) classification of HF severity is based on the symptomatology. Therefore it is crucial to directly monitor and control the main symptoms of the patients, as is suggested by this system and the present method. The system of the disclosure described below, aims to turn the most crucial symptom of heart failure, the dyspnea, into an objective quantitative set of indices that enable accurate and effective monitoring and optimization of treatment.

HF represents a major public health problem that affects close to 7 million Europeans and 5 million North Americans each year (1). Despite new and more effective pharmacological and nonpharmacological therapeutic strategies, the prognosis of patients with HF remains poor (2,3).

Because of its progressive and unstable nature, many patients develop recurrent episodes of decompensated HF, requiring frequent hospital admissions (4). Close to 1 million HF hospitalizations occur annually in the United States, with the majority of these resulting from worsening lung congestion in patients previously diagnosed with HF (5,6). There is a 30-day readmission rate of 27%, the highest among all medical conditions necessitating hospitalization (7).

Given the aging population and growing economic burden, improved management of the HF patient at home and prevention of hospital admissions have become national priorities in the US (8). The high proportion of patients that die or are readmitted within a few weeks of discharge may be related to premature discharge leading to worse outcomes and/or increased costs. Currently there are no well-defined criteria for decision making before discharging HF patients that were electively hospitalized for stabilization of deteriorating cardiac decompensation. Moreover, there are no well-defined indices that can assist in optimization of the duration and the aggressiveness of the treatment before discharge. Therefore, there is a need for more effective techniques to monitor the patients at home and to reduce the frequency admissions (9). There is also a need for more precise indices that will predict the rate of rehospitalization and will be used in the hospital before discharging the patient from the hospital, so they will gain a longer free of symptom period and a better quality of life.

Strategies to prevent cardiac decompensation include frequent physician examinations and close monitoring of the patient's clinical status to maintain optimal volume and adjust medications (10). Periodic reassessment might be useful in identifying high-risk patients if cardiocirculatory variables could be estimated accurately and noninvasively. However, currently there is no such variable:

- Clinical examination on a daily basis is impractical, except in a hospital.
- Monitoring body weight is the usual method recommended to patients to help detect incipient decompensation, but it has poor sensitivity for clinical deterioration (11).
- Brain natriuretic peptide (BNP) is a hormone that is produced and released by cardiac myocytes. Increased cardiac filling is a potent stimulus for BNP secretion, and this hormone was shown to have relevant physiologic effects in response to volume retaining, vasoconstriction, and ventricular remodeling (12). The diagnostic value of BNP and NT-proBNP is well established in patients attending the emergency room with acute dyspnea (13-14). But, frequent testing (e.g. daily) for BNP and NT-proBNP to monitor therapy for patients with CHF is not practical as overall changes require several days to become evident. Daily monitoring of BNP concentrations is unnecessary and may potentially result in misinterpretations (15).
- Remote, home, or telemonitoring using a number of relatively simple noninvasive variables such as heart rate. blood pressure, and body weight was also examined in a number of studies. Two large studies did not show a positive effect. In the American Telemonitoring to improve outcomes in HF (TELE-HF) study (16) and in the German Telemedical Intervention Monitoring in HF (TIM-HF) study (17), which together randomly assigned 2362 HF patients, no effect on HF hospitalizations or mortality was observed.

- Pulmonary congestion (PC) is an important determinant of symptoms and clinical outcomes in patients with congestive heart failure (CHF). PC can be detected by changes in intrathoracic impedance detected by implanted cardiac devices (18). Several studies previously assessed the sensitivity of the OptiVol algorithm (measuring intrathoracic impedance) to predict HF events and have reported results of sensitivity and positive predictive value in the range of 60-80% (19-21). But, the Diagnostic Outcome Trial in Heart Failure (DOT-HF trial) showed that the use of an implantable diagnostic tool to measure intrathoracic impedance with an audible patient alert did not improve outcome and increased heart failure hospitalizations and outpatient visits in heart failure patients. In other words, more information was apparently associated with worse outcomes (22,23).

The idea that decompensation of HF is preceded by a prolonged period of a subclinical slow rise of pressure or slow accumulation of fluid that can be captured by a monitoring device, allowing potential intervention, is highly attractive, but the current technologies have not proved to be so effective.

International patent application WO2013177621 discloses a cardio-pulmonary health monitoring apparatus. The apparatus comprises a contactless motion sensor configured to generate one or more movement signals representing bodily movement of a patient during a monitoring session; a processor; and a memory storing program instructions configured to cause the processor to carry out a method of processing the one or more movement signals. The method comprises extracting one or more sleep disordered breathing features from the one or more movement signals, and predicting whether a clinical event is likely to occur during a predetermined prediction horizon based on the one or more sleep disordered breathing features.

## SUMMARY OF THE INVENTION

The underlying rationale of the novel system:
The invention provides a device according to claim 1 and a method according to claim 2.

The novel device and system is based on understanding the effects of the hemodynamic changes on the lung compliance and the required respiratory work that eventually lead to the sensation of dyspnea. Cardiac decompensation leads to adaptive responses that attempt to maintain the cardiac output. The main compensatory adaptive mechanism is the increase in the preload, since cardiac output is a monotonic function of the preload, as is described by the well-established Frank-Starling law of the heart. While the normal preload of the LV is less than 12 mmHg, symptomatic patient have a preload well above 18 mmHg. The increase in the left atrial pressure (left ventricle preload) leads to backward increase in the pulmonary vain and pulmonary capillary pressures and to increase in the total amount of blood in the pulmonary system. However, the amount of blood pool in the lung has important effects on the respiratory mechanics:

- The weight of a dry lung of an adult is about 0.5 Kg, but the adult lung contains about 0.5 liter (0.5 Kg) of blood. Thus, the blood is a significant part of the lung mass, and it grows in proportion to the increase in the LV preload. Heavier lung requires an increase in the respiratory work to maintain the same ventilation.
- The lung compliance is determined by the alveolar part of the respiratory system. Each alveolus is surrounded by a dense capillary network, and this network is so extensive that it practically forms a continuous sheet of blood around each alveolus. An increase in the pressure and capillary congestion leads to "capillary erection" and a significant increase in lung stiffness (decrease in the compliance). Therefore an increase in the preload is associated with a decrease in lung compliance and an increase in the respiratory work.
- An increase in the lung blood pool increases the viscous property of the lung since the blood is incompressible but can shift between compartments within the lung. An increase in lung viscosity leads to a wider hysteresis loop in the pressure-volume plane during each breath cycle, which requires larger respiratory work. This excessive work turns into wasted heat.
- Further increase in the left atrial pressure leads to accumulation of transudative effusion in the interstitial space, within the alveolar septum. This additional pool of water further deteriorates the above lung mechanical properties: leading to additional decline in lung compliance and an increase in lung viscosity. The accumulation of fluid between the alveoli and the capillaries increases the diffusion distance between the two, leading to an impaired gas exchange. Consequently there is a need to increase lung ventilation and the respiratory work to maintain the normal blood gases. One aspect of the disclosure without limitation, is to prevent deterioration to this stage with impaired gas exchange, and to detect and prevent the progression of the disease at earlier stages.
- Further increase in the left atrial pressure and lung congestion leads ultimately to pulmonary edema with transudative effusion within the alveolar space, with the overt picture of respiratory distress.

Thus, the gradual increase in the lung blood pool progressively decreases the lung compliance and increases the viscous property of lung. These changes lead to an increase in the respiratory work required to maintain the adequate alveolar ventilation. These changes in the respiratory effort are reflected in chest wall dynamics.

To summarize, the novel technology is based on the following observations:

- Cardiac decompensation is usually associated with an increase in the preload.
- An increase in the preload increase the lung blood pool.
- An increase in the lung blood pool and the associated "capillary erection" leads to decreased lung compliance and increase lung viscosity.
- These deteriorations in lung properties (compliance and viscosity) elicit an increase in the respiratory work in order to maintain the adequate lung ventilation and blood oxygenation.

Therefore monitoring the respiratory dynamics can provide the earliest non-invasive signs for deterioration in heart failure and to the associated increase in the lung blood pool. These signs appear gradually and can be detected before the patient is aware of the increase in the respiratory effort and has no complaints of developing dyspnea.

Therefore monitoring the respiratory dynamics can provide a gamut of mechanical indices that can:

- Turn a symptom that was previously a qualitative symptom into a set of objective measurable indices.
- Detect the gradual increase in the respiratory work and provide subclinical indices, before the patients complain for dyspnea. Consequently it can assist in early detection and prevention of rehospitalization.
- Quantify the severity of the dyspnea

Consequently the system can be used for home monitoring, for optimization of medical treatment and for decision making before discharging the patients.

Quantification of the severity of dyspnea has significant clinical merit since the treatment of patients with heart failure is not simple even in the setting of the hospital. Patients that suffer from heart failure and are at NYHA class 3, are per definition hemodynamically stable at rest, and usually have reasonable and controlled heart rate and blood-pressure and excellent pulse oxymetry, close to 100%. However these patients have subjective complaints of dyspnea. These patients are hospitalized electively with symptoms of dyspnea for stabilization, but are admitted and discharged with identical hemodynamic indices. The main criteria for discharging the patients are the general physical examination and the qualitative impression of the physician from the severity of the respiratory distress, the gained decrease in weight that was induced by the various diuretics, and a semi-quantitative but subjective five-point Likert scale for dyspnea that the patients filled. The need for quantitative indices for the severity of dyspnea (which are absent in the prior art), the main cause for the admission and readmission, are clear.

We present a simple technology that is based on monitoring subclinical signs of an increase in the respiratory work, that ultimately lead to dyspnea. The patients do not notice the subclinical changes in the respiratory work and slowly adapt to the gradual changes. Some patients even disregard or deny these changes.

The technology is noninvasive, and is based on monitoring and quantifying abnormal respiratory function and pathognomonic signs deteriorating heart failure. It includes monitoring changes in the respiratory dynamics and rhythm. The decrease in lung compliance and the increase in the viscosity lead to the following pathognomonic signs:

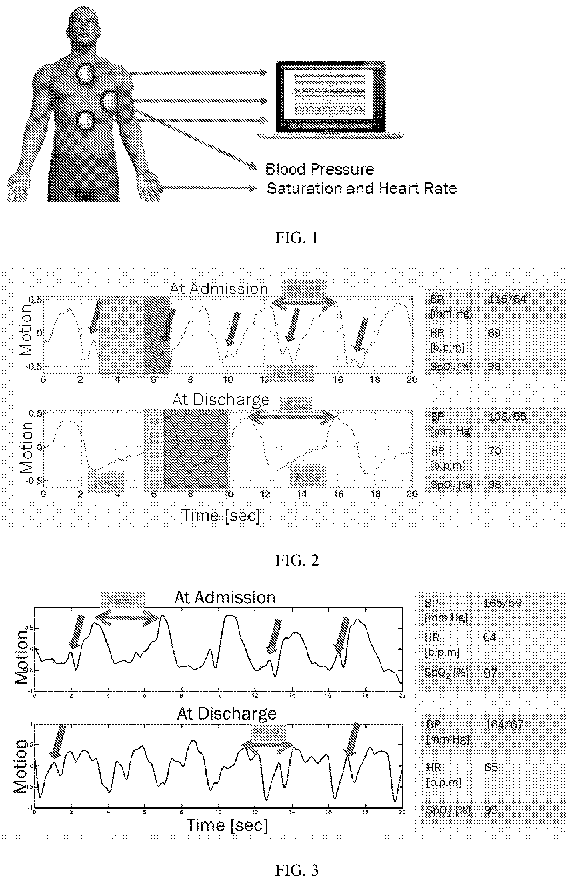

- Increase in the duration of the respiratory activity and decrease in the resting passive phase, up to the appearance of continuous periodic ventilation. This feature is quantified by the Active Duty cycle (Adc) index that depicts the fraction of the active phase out of the entire breath cycle.
- An increase in the duration of the inspiratory phase. The prolongation in the inspiratory phase may result from the increase in the work required to extend a stiffer and more viscous lung. Moreover, patients with heart failure suffer from impaired skeletal muscle activity.
- The development of an abnormal active expiratory due to the activation of accessory respiratory muscle during the expiratory phase. Interestingly this is an early sign of deteriorating heart function. The prolongation of the inspiratory phase is at the expense of the expiratory phase and therefore there is a need to expedite the expiration, as shown in Figures 2 and 3. The magnitude of this expiratory wave is proportional to the severity of the cardiac decompensation.
- The development of coarse and polyphasic inspiratory waves, due to the activation of accessory inspiratory muscles.
- The expiratory and inspiratory accessory muscle contractions yield additional waves at higher frequency and harmonics of the basic respiratory frequency. Normal breathing yields a dominant peak in the power spectrum at the basic respiratory frequency. Additional peaks at higher frequencies appear in the decompensated HF patients. An increase in the energy in these peaks, relative to the energy in the basic respiratory frequency is associated with a more severe cardiac decompensation.

- The development of periodic breathing with slow crescendo and decrescendo, where the motions represent both active inspiration and expiration. In seems that this type of ventilation is just an aggravation of the previous described features. Interestingly, it was reported that HF patients with periodic ventilation have a very poor prognosis.

The system quantifies all these features. In general an increase in the polyphasic activity that relates to the recruitment of additional accessory muscles is associated with a more severe decompensated heart failure.

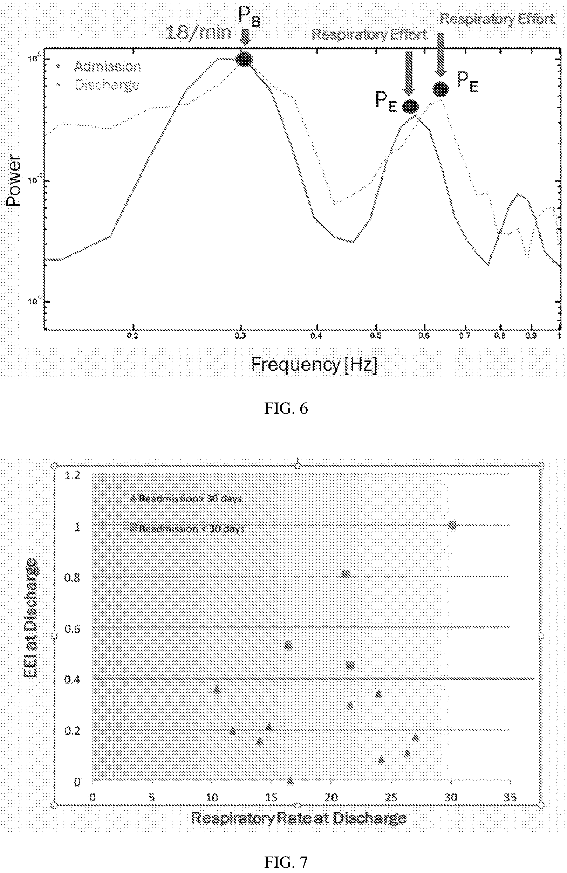

Interestingly, simple monitoring of the tidal respiratory amplitude is not pathognomonic for deteriorating heart failure, and it appears in various normal (anxiety) and pathological conditions. Monitoring the respiratory rate is also of minor added value, as is demonstrated below (Fig. 7).

It is important to note that this system is completely different in concept, indices and analyses from prior art that aims to detect short events of apneas and hypoventilation ("Apnea and hypoventilation analyzer") ( US Patents 8226571 and 8790270) and to continuously monitor the adequacy and symmetry of lung ventilation (continuous monitoring of lung ventilation, based on mechanical indices and location of the endotracheal tube). In the prior art, the main task was to quantify the changes in the ventilation. In the present disclosure for patients with decompensated heart failure, monitoring the ventilation is useless since there are no significant changes in the ventilation. Changes in the ventilation in patients with HF appear in severe conditions, as pulmonary edema, when the clinical situation is overt and the devise is useless. The cardiorespiratory center within the brainstem integrates all the chemical and hemodynamic indices, and tightly maintains the appropriate blood oxygenation. Even in severe cases, when there is already overt dyspnea, the saturation will remain normal. The brainstem activates the intrinsic and accessory respiratory muscles in order to preserve normal alveolar ventilation and blood oxygenation. Although patients with decompensated heart failure tend to have a slightly higher respiratory rate, the changes in the respiratory rate are usually not statistically significant (Fig. 7). If the patients preserve the normal pulse oxygenation, then they have adequate and normal alveolar ventilation. The minute lung ventilation is the product of the respiratory rate and the tidal volume. Therefore, one should not expect to obtain significant changes in the mean tidal volume in patients with HF. The prior art attempts to monitor and detect changes in the tidal volume and in the symmetry of lung ventilation. In patients with decompensated heart failure there are no changes in the ventilation at earlier stage, and the changes in lung mechanics are practically symmetrical. Therefore, indices as tidal chest displacement, asymmetry or asynchrony are not relevant for the monitoring heart failure patients, since the ventilation and the saturation is preserve and there is symmetrical ventilation of both lungs.

The present disclosure is based on monitoring subclinical signs of an increase in the respiratory work. There is physiological evidence that an increase in lung congestion decreases lung compliance and increases lung viscosity, and both elicit a gradual increase in the respiratory work. The patients do not notice the subclinical changes in the respiratory work, may slowly adapt to the gradual changes, or deny these changes. It is important to note that there are no significant changes in the minute ventilation or in the symmetry of ventilation at the early stages of cardiac decompensation. There are pathognomonic signs for deteriorating heart failure due to the gradual decrease in lung compliance and the associated increase in the respiratory work of the intrinsic and accessory respiratory muscle.

The technology is noninvasive, and is based on monitoring and quantifying the abnormal respiratory mechanics. In contrast to the normal breathing, which is active only during the inspiratory phase and is completely passive during the expiration, in HF the expiration is also active. Moreover, in more advance stages the inspiration and expiration are associated with coarse and polyphasic waves, due to activities of the accessory respirator muscle. The respiratory dynamics is also associated with changes in the respiratory rhythm and in severe cases there is continuous periodic breathing.

One major advantage of the invention is that it provides objective signs to the subjective symptom of dyspnea, and the feasibility was validated in the clinical study that is described below. It is important to note that patients with stage 3 and 4 heart failure rarely have normal breathing with pure passive expiration and smooth respiratory dynamics. Most of them have ongoing excessive respiratory work. Moreover, there are evidence that these patients suffer also from impaired and weak function of the respiratory muscle, since HF causes general weakness and atrophy of the skeletal muscle. Thus the sensation of dyspnea is due to both an increase in the required respiratory work and the decrease in the strength of the respiratory muscle. Moreover, the presence of ongoing excessive work and the weakness of the respiratory muscle suggest that patients with severe HR have a very low reserve and therefore they can easily suffer from dyspnea and fatigue. Therefore, a sensitive method that monitoring the earlier signs of increase in the respiratory work, and directly monitors the physiological variables that are associated with the symptoms that lead to frequent hospitalization is required.

In accordance with the present disclosure, the device has (i) the ability to provide detection of changes in the respiratory dynamics that is required for the optimization of the treatment at home. There are significant temporal variations in the pattern of the ventilation due to changes in mode and activity. To avoid false alarms, the system monitors and analyzes sequential and large time intervals (as opposed to apnea monitors, where a fast detection of apneic events within few seconds is required). (ii) The ability to quantify the changes in the respiratory dynamics that is required for the optimization of the treatment at the hospital and for making the decision before discharging the patient. The ability to quantify the severity of the excessive respiratory work, together with the general conditions of the patient, enable making predictions and defining whether the respiratory work was reduced to a reasonable level that enables the patient to remain at home for a reasonable time interval. The aim is to reduce the rate of rehospitalization within the first month of the discharge from the hospital. As shown in the study below, the excessive energy indices at discharge can predict the probability of early rehospitalization.

The system has a large potential application, since millions of patients suffer from stage 3 and 4 heart failure, and the system can improve and optimize the treatment of these patients. The system is not limited to in-hospital or at-home patient usages.

The system can alert the patient at home for progressive deterioration. It can send the information to a dedicated medical center with or without providing an alert to the patient (to have a more professional decision without causing unnecessary anxiety). It can be used for continuous monitoring of hospitalized patients with heart failure that were admitted for balancing a decompensated situation. It can serve for more precise decision before discharging a patient with heart failure. It can also be used for monitoring hospitalized heart failure patients that are at risk of cardiac deterioration and were hospitalized for other medical treatments or interventions.

There is provided in accordance with the present disclosure a device including a local acceleration sensor mountable on a chest or upper abdomen of a patient for sensing local accelerations or changes in a position, including orientation or displacement, of the local acceleration sensor, a data acquisition system and data storage for data analysis and comparison with past data, and a processor and a non-transitory, computer-readable storage medium in communication with the processor, wherein a computer-readable program code is embodied in the storage medium, and wherein the computer-readable program code is configured to classify phases of a respiratory cycle, including inspiration and expiration phases, and polyphasic motions within the phases, to calculate energy of the polyphasic motions based on sensed information of the local acceleration sensor and to classify severity of cardiac decompensation by calculating an excessive energy index (EEi) that compares excessive energy that appears in the polyphasic motions to energy required for inspiration at a basic respiratory rate.

In accordance with the invention the excessive energy index (EEi) includes a ratio between a sum of the energies of the polyphasic motions at high order harmonics of basic respiratory frequencies and all energy at a frequency higher than a basic respiratory frequency, to the energy at the basic breathing frequency, at a measurement site of the local acceleration sensor.

In accordance with an embodiment, that is not part of the invention, the excessive energy index (EEi) is calculated based on an excessive respiratory peak that is pathognomonic to decompensated heart failure and the EEi includes a ratio between an amplitude or energy of the excessive respiratory peak and an amplitude or energy at an inspiratory wave, at a measurement site of the local acceleration chest sensor.

In accordance with an embodiment, that is not part of the invention, the EEi includes an Activity Duty Cycle (Adc), which includes a ratio of duration of a vigorous breath activity to an entire duration of breath cycle, the vigorous breath activity being defined as a breath activity during a portion of the breath cycle which is more vigorous than other breath activity in other portions of the breath cycle.

There is provided in accordance with the present disclosure a method including sensing local accelerations or changes in sensor position, including orientation and displacement, with a local acceleration sensor mounted on a chest or abdomen of a patient, and calculating energy of polyphasic motions, based on sensed information of the local acceleration sensor and classifying severity of cardiac decompensation by calculating an excessive energy index (EEi) that compares excessive energy that appears in the polyphasic motions to energy required for inspiration at a basic respiratory rate.

The method may include using local acceleration sensors, one placed on an upper sternum, close to a suprasternal notch, another placed on a left side of the cheat at a region of cardiac point of maximal impact (PMI) and another placed on a upper abdomen (epigastrium) near a diaphragm of the patient, and determining different polyphasic activities at these different sites. The sternal and epigastric sensors are sensitive to the activity of the accessory respiratory muscles at the neck and the abdomen, respectively. The sensor the PMI area is sensitive to changes in the cardiac impact or to development of additional cardiac sounds or vibrations.

BRIEF DESCRIPTION OF THE DRAWINGS

The present invention will be understood and appreciated more fully from the following detailed description, taken in conjunction with the drawings in which:

Figure 1 is a simplified illustration of a device for monitoring and patients with heart failure, constructed and operative in accordance with an embodiment of the present disclosure. The system detects excessive work of accessory respiratory muscle at the neck, which are very important for fixing the chest cage (detected by the sensor at the supra sternal notch), or the abdominal accessory muscle (detected by the sensor at the epigastrium). It can also monitor changes in the impact of the heart and the heart sounds and vibration (utilizing the sensor at the PMI - point of maximal impact). Consequently it may detect also the heart rate, as an adjuvant index.

**Figure 2** is an example of successful treatment of patient with decompensated heart failure that was electively admitted for hospitalization with a leading symptom of dyspnea. This patient had been treated with diuretics and his clinical condition was balanced, and he was readmitted only after 90 days.

The hemodynamic indices (BP=blood pressure, HR-Heart rate), the pulse oximetry ($SpO_2$) (on the right hand side) together with the calculated respiratory motion (derived by the algorithm of the system) at admission (upper part) and discharge (the bottom). Note that there were no changes in these parameters, and they were normal already at admission. On admission the respiratory rate was slightly elevated (17 bpm), but what was more prominent is the continuous and restless breathing, without a moment of rest (quantified by the Adc index). Respiratory rate decreased from admission to discharge (from 17 to 12 bpm), but more significantly, the decrease in the rate is due to the appearance of almost normal and prolonged period of rest during the expiratory phase (normal and low Adc). The most significant phenomenon in this example was a polyphasic and active expiration that was observed at admission (arrows) which diminished towards discharge (was quantified by the EEi indices). The arrows show the additional polyphasic expiratory movement at admission. Interestingly, there are also significant changes in the relative duration of the inspiratory (green) and expiratory (gray) phases. At admission the patient has significant and abnormal prolongation of the inspiratory phase, due to the decrease in the lung compliance. At discharge the ratio between these two phases is practically normal.

**Figure 3.** The figure shows signals of a patient to whom the treatment did not help; he was re-hospitalized due to HF decompensation only 11 days post discharge. This patient has been treated with diuretics and his clinical condition, according to the novel system recording, was not balanced. The system predicted that the treatment was unsuccessful, and that the patient was discharged with severe signs of excessive respiratory work.

There were no significant changes in the following parameters: hemodynamic indices (BP=blood pressure, HR-Heart rate), the pulse oximetry ($SpO_2$) (on the right hand side) together with the calculated respiratory motion (derived by the algorithm of the system) at admission (upper part) and discharge (the bottom). On admission respiratory rate was elevated (20 bpm). The respiratory rate increases from admission to discharge (from 20 to 30 bpm), but more significantly, there was an increase in the intensity of the active motion with various polyphasic waves during the inspiration and expiration (An increase in the Adc index). The arrows depict the appearance of prominent active expiratory movements that were observed at admission (arrows) and discharge (was quantified by the EEi indices).

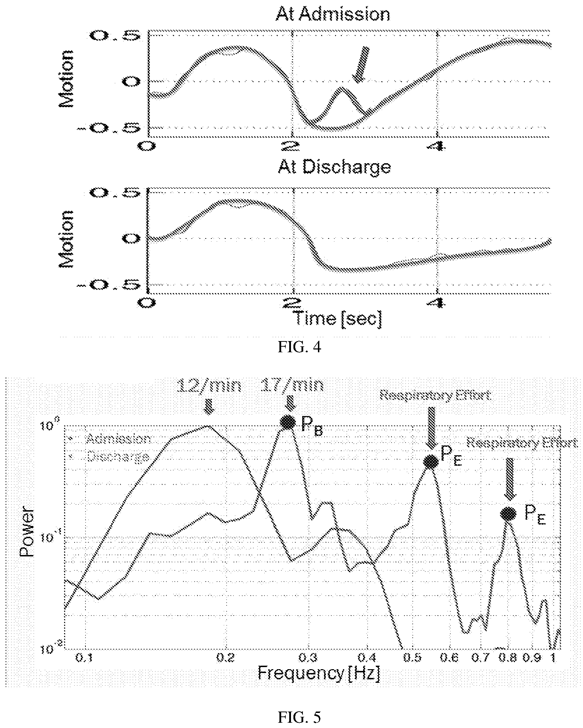

**Figure 4.** Heart failure patients have a pathognomonic sign of excessive expiratory work. Real respiratory patterns on admission (upper part) in a state of HF decompensation on admission and discharge (lower part). On admission there is an additional wave during the expiration (the "humped" curve pointed by the arrow) which indicates an additional increase of extra respiratory effort. It is used as a novel index to monitor the severity of congestion of heart failure patients.

**Figure 5.** The power spectral analyses on admission (the graph that continues to the right) and discharge (the graph with the peak at 12/min) from a patient that was successfully treated for decompensated heart failure. The power spectral analysis shows the energy distribution of patient respiration. Most of the energy is concentrated around the basic breathing rate (denoted by $P_B$). However at admission there were additional significant peaks at higher frequencies (denoted by $P_E$). They represent the additional respiratory work during the expiratory phase (Figure 3) and the other polyphasic motions due to the various accessory respiratory muscle activities, also during the inspiratory phase. At discharge the excessive work at the higher frequencies disappeared and there was only one significant peak of energy, at the patient natural breathing rate. This patient was not re-hospitalized during the 30 days post hospital discharge.

**Figure 6.** The power spectral analyses on admission (the graph that starts with a lower y value) and discharge (the other graph) from a patient that did not respond to the diuretic treatment according to our system that monitor the severity of dyspnea, and was re-hospitalized within the 30 days post hospital discharge period. The figure depicts the power spectral analyses on admission and discharge. The power spectral analysis shows the energy distribution of patient respiration. Most of the energy is concentrated around the basic breathing rate (denoted by $P_B$). However at admission there were additional significant peaks at higher frequency (denoted by $P_E$). This additional peak represents the additional respiratory work during the expiratory phase (Figure 3) and the other polyphasic motions due to the various accessory respiratory muscle activities, also during the inspiratory phase. Interestingly, at discharge the excessive work at this higher frequency increased from about 30% of the amplitude of the peak at the basic respiratory rate ($P_B$) to 45% of this energy, suggesting that was a significant aggravation in the excessive respiratory work.

**Figure 7.** The figure depicts the utility of the Excessive Energy index (EEI) and the respiratory rate in differentiating between patients that be re-hospitalized within less than 30days those that will gain a prolonged free of symptom period. The group of patients that were free from a HF re-hospitalization during the first thirty days post discharge (triangles) had EEI value below 0.4 at discharge. While all the patients that were readmitted within the first month post hospital discharge (squares) had EEI value above 0.4. Interestingly, the respiratory rate was inconsistent and varied substantially in a wide range of frequencies, and could not differentiate between the two groups.

DETAILED DESCRIPTION OF EMBODIMENTS

Reference is now made to Fig. 1, which illustrates a device for monitoring the respiratory dynamics for detecting and quantifying the development of signs for excessive respiratory work that is pathognomonic to HF. The system operates in accordance with a non-limiting embodiment of the present disclosure.

The device can continuously acquire and analyze the respiratory dynamics for a single sensor on the chest or the abdomen. In one embodiment, the sensor is an accelerometer. Other sensors, such as gyro-meters or gyroscope, which sense and quantify the motion of a single point in the space, can be used (all these types of sensors are referred to as a local acceleration sensor). Other accelerometers or alike may be used, such as on the abdomen. The sensors may be embodied in a patch, referred to as a patch and sensor unit.

The system includes

- the hardware for data amplification and filtration.
- the required data acquisition system and data storage for continuous data analysis and comparison with the past.
- The system includes a processor (central processing unit), which may include additional channels for ECG acquisition with appropriate gain and filtering.

Software for analyzing the pathognomonic signs of progressing HF (in other words, the processor uses a non-transitory, computer-readable storage medium, and computer-readable program code is embodied in the storage medium, wherein the computer-readable program code is configured for analyzing the pathognomonic signs of progressing HF).

- User-friendly interface and display of the new indices for the excessive respiratory energy.

The system may also include contact electrode(s) for ECG, which may be in each patch and sensor unit.

All the sensors are connected to processor. The processor analyzes the sensed local accelerations and\displacement and\or motion of each sensor and quantifies the severity of the above mentioned indices for the excessive respiratory work.

The following is noted:

(i) Measurement of the acceleration provides the following advantages:

1. Enables assessment of the displacement and acceleration of a single anatomic point of interest.
2. Provides the position of the sensor in space, utilizing the gravity acceleration.
3. Flat frequency response form DC to the high sound frequency. Therefore it can detect the subsonic respiratory dynamics.
4. Miniature sensor, with low energy consumption.
5. The measured acceleration is proportional to the forces that operate on the mass to which the sensor is attached, assuming that the mass is constant.

(ii) Frequent hospitalizations due to decompensated HF are due to the development of lung congestion. Although lung congestion is associated with changes in the lung compliance and lung viscosity, there is no prior art device to monitor the changes in lung mechanical characteristic with the development of progressing HF.

(iii) Patients with mild to moderate decompensated HF have normal saturation and adequate lung ventilation. To maintain the adequate lung ventilation the increase the respiratory work and utilize also the accessory respiratory muscles.

(iv) Increase in the respiratory work can develop in various normal (anxiety) and pathological (as airway obstruction). However, decompensated heart failure is associated with pathognomonic feature, since also the underlying changes in lung characteristics are quite unique. Decompensated heart failure is associated with a decrease in the lung compliance and an increase in lung viscosity. Upper airways obstruction is associated with a simple increase in the resistance to flow. COPD is associated with an increase in lung compliance (opposite to HF) and an increase in the resistance at the lower airways level and mainly during the expiratory phase. These differences in the affected components (resistance vs. compliance) and lung compartments (upper airways vs. lower airways) are associated with different transfer functions and observed phenomena. The above described features are pathognomonic for HF and thus different indices should be analyzes and monitored in decompensated HF.

Study protocol

The study enrolled patients who arrived at the hospital with decompensated heart failure. On admission and discharge, the patients had measurements of blood pressure, pulse rate, oxygen saturation, and weight, and underwent a physical examination. Blood level of BNP was measured on admission. At hospital discharge the patients filled a Likert scale questionnaire, as a subjective indicator to the improvement in respiratory effort. Three motion sensors, which record the motion signals, were placed on the chest and over the epigastric area of the patient. The patients were monitored close to hospital admission and at the day of discharge, each time for a period of about thirty minutes. During the monitoring period patient's hemodynamic indices were taken.

Sensors placement

The sensors were placed in 3 locations:
One sensor was placed on the upper sternum below the supra-sternal notch in order to detect excessive work of the muscles of the neck. This sensor is also very sensitive to breathing.
A second sensor was placed near the cardiac apex at the point of maximal impact (PMI) in order to detect changes in the cardiac impact and heart sounds. The sensor is sensitive to signals originated from the heart.
The third sensor placed at the epigastrium is sensitive to the motion of the diaphragm and the accessory abdominal respiratory muscle. It detects excessive work of the abdominal muscles.

Experimental setup

The system we used comprises of three, miniature, two axial accelerometers, connected to a data acquisition unit.

Data analysis

Data were acquired at a sampling rate of 5 KHz and analyzed offline with a specialized analysis program implemented in MATLAB (Mathworks) under the scope of the study. Results of all experiments were summarized for statistical analysis.

Signals pre-processing

The sampled data carried noise originated by patients' movement and talking. Since we were interested in the respiratory dynamics, the data was down-sampled to 500Hz.

Signal Phase Estimation

Both the longitudinal and radial axis carry important information about the breathing signal, therefor it is important to combine the contributions from both axis.

Clinical Data

We compare the respiratory signals of a patient at admission and discharge. We found several prominent differences between the signal from admission and discharge:
In most of the patient the respiratory rate decreases on discharge compared to admission, as shown in Figures 2 and 5. In few patients there was a slight increase in the respiratory rate.
On admission the breathing is continuous and restless with no quiescent phases during respiration, as shown in Fig 2. On the other hand, at discharge, there are phases of rest during the late expiratory phase in the majority of the cases. When the patients are discharged with prominent and continuous active breathing they are readmitted for hospitalization within less the 30 days (Figure 3)
The most significant phenomenon is a polyphasic and active expiration seen at admission and diminishes towards discharge. We document for the first time that decompensated heart failure patients increase respiratory work during the expiration phase by recruiting abdominal respiratory muscles as shown in Figures 2 and 4.

Excessive effort index

In normal condition expiration should be passive. However, we found in decompensated state, the respiratory signal is characterized by active and polyphasic expiratory phase. This phenomenon is depicted as additional movement or harmonic to the basic breath cycle. Therefore, it will be manifested as additional energy concentration at higher frequency harmonics in the signal spectrum.

We use Welch's method to estimate the power spectral density. Welch's method is based on averaging the periodogram. The periodogram is a nonparametric estimate of the power spectral density (PSD) of a wide- sense stationary random process.

Welch's technique to reduce the variance of the periodogram breaks the time series into overlapped segments. Welch's method computes a periodogram for each segment and then averages these estimates to produce the estimate of the power spectral density. Because the process is wide-sense stationary and Welch's method uses PSD estimates of different segments of the time series, the periodograms represent approximately uncorrelated estimates of the true PSD and averaging reduces the variability.

Energy calculation

First, the signal is segmented into overlapping windows. The PSD resolution depends on the window length by:

$$RES_{PSD} = \frac{F_S M}{2 N_{window}} \ [Hz]$$

Where $F_S$ is the sampling frequency, M is the number of windows Welch's method breaks the signal to, as was explained in the previous section. Usually M=8. N is the length of the window in to what we segment the signal. In order to maintain a PSD resolution of 1/60 Hz (1 bpm) with $F_S$ of 500 [Hz], we need to choose a window length of at least 270 [sec]. That means that the periodogram will be performed to 30 [sec] signals, with 50% overlap and we can assume that the signals are stationary. Then, for each window, the PSD is evaluated. The highest energy concentration is found around the natural breathing rate, we consider this to be the breathing frequency-$F_b$. The breathing energy is defined as:

$$E_{Br} = \frac{\sqrt{2}}{f_b} \sum_{f=\frac{1}{\sqrt{2}}f_b}^{\sqrt{2}f_b} P_{xx}(f)$$

In the analysis presented here the excessive energy at high frequency is calculated as:

$$E_{Ex} = \frac{1}{1-\sqrt{2}f_b} \sum_{f=\sqrt{2}f_b}^{1} P_{xx}(f)$$

Where $f_b$ is the breathing frequency and $P_{xx}$ is the power spectral density values. The Excessive Energy index (EEI) is defined as the ratio between the excessive energy and the energy at the basic breathing frequency (respiratory rate):

$EEI = E_{Ex} / E_{Br}$

In healthy subjects the EEI should be zero and it increase with the increase in the appearance of excessive work.

Respiratory Rate

The respiratory rate estimation process consists of several steps. First, to remove all the unwanted information, which is above patient breathing frequency, the signal was filtered using a low pass filter with cutoff frequency of 1 [Hz]. To maintain stationarity, we segment the signal into thirty seconds windows with an overlap of fifty percent between them. For every window we calculate the Hilbert transform and find its peaks.

One of the properties of Hilbert transform is that it is an odd function. That means that it will cross zero on the x-axis every time that there is an inflection point in the original waveform. Similarly a crossing of the zero between consecutive positive and negative inflection points in the original waveform will be presented as a peak in its Hilbert transformed conjugate.

RESULTS

Changes in EEI from Admission to Discharge

Readmission after a hospitalization for heart failure is an important target for quality improvement. There is a high thirty-day readmission rate (30%). Recognition of the importance of readmissions as a measure of quality is still very recent. Early readmission within the first thirty days post discharge is considered as treatment failure. Several strategies to lower hospital readmission rate for patients with heart failure had been developed (24).

The study group included 14 patients (Table 1). All had severe stage 3 or 4 heart failure. We divide the patients into 2 groups: responders and non-responders. The responders (n=10) had free of hospitalization period longer than 30 days, and the non-responders (n=4) were re-hospitalized within less than 30 days. There was no significant difference between the two groups in their age (71.4±9.8 vs. 73.8±8.3 years), cardiac ejection-fraction (24.4±9.8% vs. 25.2±7.1%) of BNP levels (1843±1461 vs. 1489±121). Interestingly. There was also no significant difference between the two groups in the hemodynamic indices: heart rate (74±10 vs. 77±15 bpm), or systolic pressure (121±14 vs. 126±54 mmHg) of diastolic pressure (Table 1). Not surprisingly, the pulse oxymetry were normal and identical in the two groups, since they were normal also at admission. Thus the hemodynamic and the saturation can't differentiate between the two groups. The responder have a slightly larger decrease in the weight than the non-responder (3.75±1.65 vs. 2.5±1.3 Kg), however this difference was not statistically significant. The responder have also a slightly larger decrease in the respiratory rate than the non-responder (3.8±3.6 vs. 1.1±4.3 bpm), however this difference was also not statistically significant. Only the EEI could differentiate between the two groups (p<0.01). In the responder group there was a significant decrease of 45% in the EEI, while the in the non-responder there was even an increase of 23% in the EEI.

Moreover, when we plot the EEI index against the respiratory rate (Fig. 7) we can see that there is a clear separation between the responders (triangles) and the non-responders (rectangular). In the responder group the EEI at discharge was below 0.4. While in the non-responders it was above 0.4. In both groups, the respiratory rate, was inconsistent and varied substantially in wide range of frequencies.

Figure 7 also verifies that the respiratory rate can't assist in the differentiation between the two groups, since there was a clear overlap between the two groups in the distributions of the respiratory rate at discharge (Fig. 7).

Discussion

For the first time, we documented that HF decompensation is associated with increased respiratory work during the expiration phase. HF decompensation leads to decrease in lung compliance and increase in the viscous loads, that leads to increase of the respiratory work and was associated with appearance of dyspnea.

Chronic patients adapt to their physiological condition and may preserve the required cardiac output by elevating the cardiac filling pressures. However, the increase in the filling pressure and the associated increase in the lung blood pool, further decrease lung compliance, leading to an increase in the respiratory work and to a sensation of dyspnea.

We have established a novel index (EEI) that can be used for assessing the severity of the excessive respiratory work, and it also correlates with the severity of the dyspnea. We have turned the subjective symptom of dyspnea into a measureable objective and absolute marker, the EEI that is proportional to the severity of congestion.

In contradiction to what might be expected, the BNP levels in the responder group were higher than the BNP levels in the non-responder group. However, it is important to note that these BNP levels were collected at admission, and the difference was not statistically significant.

In both groups there was a significant reduction in weight and respiratory rate during the hospitalization. However, there was no difference between the two groups at discharge. These observations strengthen the fact that the decreases in the weight or respiratory rate have low predictive values. EEI was the only statistically significant parameter that enabled us to differentiate between the two groups of patients. Therefore we suggest that the EEI should be used for decision making before discharging the patients from the hospital.

| | Long Phase (vent period) [n=18] | Early Readmission [<60 day] [n=9] | |
|---|---|---|---|
| Age [years] | 71.4±9.8 | 73.8±8.3 | |
| EF [%] | 24.4±9.8 | 25.2±7.1 | |
| BNP [pg/ml] | 1843±1461 | 1489.6±1213 | |
| Heart Rate [bpm] | 74±10 | 77±15 | |
| Blood Pressure Systolic [mm Hg] | 121±14 | 126±65 | |
| Blood Pressure Diastolic [mm Hg] | 73±11 | 68±13 | |
| $SpO_2$ [%] | 97±2 | 97±1 | |
| Δ Weight [Kg] | -3.75±1.65 | -2.5±1.3 | |
| Δ respiratory Rate [bpm] | -3.8±3.6 | -1.13±4.3 | |
| EEI Index | -0.45±0.32 | +0.23±0.25 | P<0.01 |

Table 1. Summary of the accumulated results of the clinical study.

Bibliography:

1. Stewart S. Financial aspects of heart failure programs of care. Eur J Heart Fail. 2005;7:423- 428.

2. Cleland JG, et al. The effect of cardiac resynchronization on morbidity and mortality in heart failure. N Engl J Med. 2005;352:1539 -1549.

3. Pfeffer MA, et al. CHARM Investigators and Committees. Effects of candesartan on mortality and morbidity in patients with chronic heart failure: the CHARM-Overall programme. Lancet. 2003;362:759 -766.

4. Vanderheyden M, et al. Continuous Monitoring of Intrathoracic Impedance and Right Ventricular Pressures in Patients With Heart Failure. Circ Heart Fail. 2010; 3:370-377.

5. Roger VL, et al. Heart disease and stroke statistics--2011 update: a report from the American Heart Association. Circulation. 2011; 123:e18-e209.

6. Bui AL, et al. Home Monitoring for Heart Failure Management. Am Coll Cardiol. 2012; 59(2): 97-104.

7. Jencks SF, et al. Rehospitalizations among patients in the Medicare fee-forservice program. N Engl J Med. 2009; 360:1418-1428.

8. Lee WC, et al. Economic burden of heart failure: a summary of recent literature. Heart Lung. 2004; 33:362-371.

9. Louis AA, et al. A systematicr eview of telemonitoring for the management of heart failure. Eur J Heart Fail 2003;5:583-590.

10. Fonarow GC, et al. Impact of a comprehensive heart failure management program on hospital readmission and functional status of patients with advanced heart failure. J Am Coll Cardiol 1997;30:725-32.

11. Lewin J, et al. Clinical deterioration in established heart failure: what is the value of BNP and weight gain in aiding diagnosis? Eur J Heart Fail 2005;7:953-7.

12. Maisel AS, et al. Cardiac natriuretic peptides: a proteomic window to cardiac function and clinical management. Rev Cardiovasc Med. 2003;4:S3-S12.

13. Maisel AS, et al. Rapid measurement of B-type natriuretic peptide in the emergency diagnosis of HF. N Engl J Med 2002;347:161-7.

14. [9] Bayes-Genis A, et al. N-terminal probrain natriuretic peptide (NT-proBNP) in the emergency diagnosis and in-hospital monitoring of patients with dyspnoea and ventricular dysfunction. Eur J Heart Fail 2004;6:313-8.

15. Wu Alan H.B, et al. Biological variation of the natriuretic peptides and their role in monitoring patients with heart failure. Eur J Heart Fail 2004;6:355-8.

16. Chaudhry SI, et al. Telemonitoring in patients with heart failure. N Engl J Med. 2010;363:2301-2309.

17. Koehler F, et al, on behalf of the TIM-HF Investigators. Impact of remote telemedical management on mortality and hospitalizations in ambulatory patients with chronic heart failure: the telemedical interventional monitoring in heart failure study. Circulation. 2011;123:1873-1880.

18. Heist EK. Et al. for the DEFEAT-PE Study Investigators. Analysis of Different Device-Based Intrathoracic Impedance Vectors for Detection of Heart Failure Events (from the Detect Fluid Early from Intrathoracic Impedance Monitoring Study). Am J Cardiol 2014;114:1249-1256.

19. Yu CM, et al. Intrathoracic impedance monitoring in patients with heart failure: correlation with fluid status and feasibility of early warning preceding hospitalization Circulation 2005;112:841-848.

20. Vollmann D, et al. Clinical utility of intrathoracic impedance monitoring to alert patients with an implanted device of deteriorating chronic heart failure. Eur Heart J 2007;28:1835-1840.

21. Abraham WT, et al. On behalf of the FAST investigators. Superior performance of intrathoracic impedance-derived fluid index versus daily weight monitoring in heart failure patients: results of the Fluid Accumulation Status Trial (FAST).J Card Fail 2009;15:813.

22. Van Veldhuisen DJ, et al. Intrathoracic Impedance Monitoring, Audible Patient Alerts, and Outcome in Patients With Heart Failure. Circulation. 2011;124:1719-1726.

23. Udelson JE, et al. T.M.I. (Too Much Information)? Circulation. 2011;124:1697-1699.

24. Bradley EH, et al. Hospital Strategies Associated With 30-Day Readmission Rates for Patients With Heart Failure. Circ Cardiovasc Qual Outcomes. 2013; 6:444-450.

## Claims

1. A device comprising:

    a local acceleration sensor configured to be mounted on a chest or upper abdomen of a patient for sensing local accelerations or changes in a position, including orientation or displacement, of the local acceleration sensor;
    a data acquisition system and data storage for data analysis and comparison with past data; and
    a processor and a non-transitory, computer-readable storage medium in communication with the processor, wherein a computer-readable program code is embodied in the storage medium, and wherein the computer-readable program code is configured to segment into overlapping time intervals the sensed information of said local acceleration sensor, and, based on analyzing the segmented overlapping time intervals of sensed information of said local acceleration sensor, determine a basic breathing frequency ($f_b$), and calculate an energy index (EEi) that compares energy appearing at frequencies higher than the basic breathing frequency ($f_b$) to energy required for inspiration at the basic breathing frequency ($f_b$) by

        - calculating a ratio ($E_{ex}/E_{br}$) between the a sum of the energies in the frequency band between $\sqrt{2}*f_b$ and

1 ($E_{ex}$), to the sum of energies in the frequency band between (1 /√2)*$f_b$ and √2*$f_b$ ($E_{br}$), at a measurement site of said local acceleration sensor; wherein the energy index is defined as the calculated ratio.

**2.** A method comprising:

sensing local accelerations or changes in sensor position, including orientation and displacement, with a local acceleration sensor mounted on a chest or upper abdomen of a patient, using a computer-readable program code, segmenting into overlapping time intevals the sensed information of said local acceleration sensor; based on analyzing the segmented overlapping time intervals of sensed information of said local acceleration sensor, determining a basic breathing frequency ($f_b$), and calculating an energy index (EEi) that compares energy appearing at frequencies higher than the basic breathing frequency ($f_b$) to energy required for inspiration at the basic breathing frequency ($f_b$) by

- calculating a ratio ($E_{ex}/E_{br}$) between the sum of the energies in the frequency band between √2*$f_b$ and 1 ($E_{ex}$), to the sum of energies in the frequency band between (1 /√2)*$f_b$ and √2*$f_b$ ($E_{br}$), at a measurement site of said local acceleration sensor; wherein the energy index is defined as the calculated ratio.

**Patentansprüche**

**1.** Vorrichtung, umfassend:

einen lokalen Beschleunigungssensor, der konfiguriert ist, um auf einer Brust oder einem Oberbauch eines Patienten zum Erfassen von lokalen Beschleunigungen oder Änderungen der Position, unter Einschluss der Ausrichtung oder der Verschiebung des lokalen Beschleunigungssensors angebracht ist, ein Datenerfassungs-System und Datenspeicherung zur Datenanalyse und zum Vergleich mit früheren Daten; und einen Prozessor und ein nicht transitorisches, computerlesbares Speichermedium in Kommunikation mit dem Prozessor, wobei ein computerlesbarer Programmcode im Speichermedium eingebettet ist und wobei der computerlesbare Programmcode zum Segmentieren der erfassten Informationen des genannten lokalen Beschleunigungssensors in überlappende Zeitintervalle konfiguriert ist, und auf der Grundlage des Analysierens der segmentierten überlappenden Zeitintervalle von erfassten Informationen des genannten lokalen Beschleunigungssensors Bestimmen einer grundlegenden Atemfrequenz ($f_b$) und Berechnen eines Energie-Indexes (EE$_i$), der Energie, die in Frequenzen auftritt, die höher sind als die grundlegende Atemfrequenz ($f_b$), mit der Energie vergleicht, die zum Einatmen in der grundlegenden Atemfrequenz ($f_b$) erforderlich ist, Berechnen eines Verhältnisses ($E_{ex}/E_{br}$) zwischen der Summe der Energien im Frequenzband zwischen √2*$f_b$ und 1 ($E_{ex}$) zur Summe von Energien im Frequenzband zwischen (1/√2*$f_b$ und √2*$f_b$ ($E_{br}$) an einem Mess-Standort des genannten lokalen Beschleunigungssensors, wobei der Energie-Index als das berechnete Verhältnis definiert ist.

**2.** Verfahren, umfassend:

Erfassen lokaler Beschleunigungen oder Änderungen in der Sensorposition, unter Einschluss der Ausrichtung und der Verschiebung, mit einem lokalen Beschleunigungssensor, der auf einer Brust oder einem Oberbauch eines Patienten angebracht wird, Verwenden eines computerlesbaren Programmcodes, Segmentieren der erfassten Informationen des genannten Beschleunigungssensors in überlappende Zeitintervalle auf der Grundlage des Analysierens der segmentierten überlappenden Zeitintervalle von erfassten Informationen des genannten lokalen Beschleunigungssensors und Bestimmen einer grundlegenden Atemfrequenz ($f_b$) und Berechnen eines Energie-Indexes (EE$_i$), der die Energie, die bei Frequenzen auftritt, die höher sind als die grundlegende Atemfrequenz ($f_b$), mit der Energie vergleicht, die zum Einatmen bei der grundlegenden Atemfrequenz ($f_b$) erforderlich ist, Berechnen eines Verhältnisses ($E_{ex}/E_{br}$) zwischen der Summe der Energien im Frequenzband zwischen √2*$f_b$ und 1 ($E_{ex}$) zu der Summe von Energien im Frequenzband zwischen (1/√2) * $f_b$ und √2 * $f_b$ ($E_{br}$) an einem Mess-Standort des genannten lokalen Beschleunigungssensors, wobei der Energie-Index als das berechnete Verhältnis definiert wird.

**Revendications**

1. Dispositif comprenant :

   un capteur d'accélérations locales configuré pour être monté sur une poitrine ou un abdomen supérieur d'un patient pour détecter des accélérations ou modifications locales sur une position, comportant l'orientation ou le déplacement, du capteur d'accélérations locales ;
   un système d'acquisition de données et un stockage de données pour l'analyse de données et la comparaison avec des données passées ; et
   un processeur et un support de stockage non-transitoire lisible par ordinateur en communication avec le processeur, dans lequel un code de programme lisible par ordinateur est mis en œuvre dans le support de stockage, et dans lequel le code de programme lisible par ordinateur est configuré pour segmenter en intervalles de temps de chevauchement les informations détectées dudit capteur d'accélérations locales et, sur la base de l'analyse des intervalles de temps de chevauchement segmentés des informations détectées dudit capteur d'accélérations locales, déterminer une fréquence respiratoire de base ($f_b$), et calculer un indice énergétique (EEi) qui compare l'énergie apparaissant à des fréquences supérieures à la fréquence respiratoire de base ($f_b$) à l'énergie requise pour l'inspiration à la fréquence respiratoire de base ($f_b$) en
   calculant un rapport ($E_{ex}/E_{br}$) entre la somme des énergies dans la bande de fréquences entre $\sqrt{2}*f_b$ et 1($E_{ex}$), sur la somme d'énergies dans la bande de fréquences entre (1/$\sqrt{2}$)*$f_b$ et $\sqrt{2}*f_b$ ($E_{br}$), sur un site de mesure dudit capteur d'accélérations locales ; dans lequel l'indice énergétique est défini comme le rapport calculé.

2. Procédé comprenant :

   détecter des accélérations ou modifications locales sur une position de capteur, comportant l'orientation et le déplacement, avec un capteur d'accélérations locales monté sur une poitrine ou un abdomen supérieur d'un patient,
   utiliser un code de programme lisible par ordinateur, segmentant en intervalles de temps de chevauchement les informations détectées dudit capteur d'accélérations locales ; sur la base de l'analyse des intervalles de temps de chevauchement segmentés des informations détectées dudit capteur d'accélérations locales ; et
   déterminer une fréquence respiratoire de base ($f_b$), et calculer un indice énergétique (EEi) qui compare l'énergie apparaissant à des fréquences supérieures à la fréquence respiratoire de base ($f_b$) à l'énergie requise pour l'inspiration à la fréquence respiratoire de base ($f_b$) en
   calculant un rapport ($E_{ex}/E_{br}$) entre la somme des énergies dans la bande de fréquences $\sqrt{2}*f_b$ et 1($E_{ex}$), sur la somme d'énergies dans la bande de fréquences entre (1/$\sqrt{2}$)*$f_b$ et $\sqrt{2}*f_b$ ($E_{br}$), sur un site de mesure dudit capteur d'accélérations locales ; dans lequel l'indice énergétique est défini comme le rapport calculé.

FIG. 1

FIG. 2

FIG. 3

At Admission

At Discharge

FIG. 4

12/min    17/min    Respiratory Effort

Respiratory Effort

$P_B$

$P_E$

$P_E$

Admission
Discharge

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013177621 A
- US 8226571 B
- US 8790270 B

**Non-patent literature cited in the description**

- **STEWART S.** Financial aspects of heart failure programs of care. *Eur J Heart Fail.,* 2005, vol. 7, 423-428
- **CLELAND JG et al.** The effect of cardiac resynchronization on morbidity and mortality in heart failure. *N Engl J Med.,* 2005, vol. 352, 1539-1549
- **PFEFFER MA et al.** CHARM Investigators and Committees. Effects of candesartan on mortality and morbidity in patients with chronic heart failure: the CHARM-Overall programme. *Lancet,* 2003, vol. 362, 759-766
- **VANDERHEYDEN M et al.** Continuous Monitoring of Intrathoracic Impedance and Right Ventricular Pressures in Patients With Heart Failure. *Circ Heart Fail.,* 2010, vol. 3, 370-377
- **ROGER VL et al.** Heart disease and stroke statistics--2011 update: a report from the American Heart Association. *Circulation,* 2011, vol. 123, e18-e209
- **BUI AL et al.** Home Monitoring for Heart Failure Management. *Am Coll Cardiol.,* 2012, vol. 59 (2), 97-104
- **JENCKS SF et al.** Rehospitalizations among patients in the Medicare fee-forservice program. *N Engl J Med.,* 2009, vol. 360, 1418-1428
- **LEE WC et al.** Economic burden of heart failure: a summary of recent literature. *Heart Lung.,* 2004, vol. 33, 362-371
- **LOUIS AA et al.** A systematicr eview of telemonitoring for the management of heart failure. *Eur J Heart Fail,* 2003, vol. 5, 583-590
- **FONAROW GC et al.** Impact of a comprehensive heart failure management program on hospital readmission and functional status of patients with advanced heart failure. *J Am Coll Cardiol,* 1997, vol. 30, 725-32
- **LEWIN J et al.** Clinical deterioration in established heart failure: what is the value of BNP and weight gain in aiding diagnosis?. *Eur J Heart Fail,* 2005, vol. 7, 953-7

- **MAISEL AS et al.** Cardiac natriuretic peptides: a proteomic window to cardiac function and clinical management. *Rev Cardiovasc Med.,* 2003, vol. 4, S3-S12
- **MAISEL AS et al.** Rapid measurement of B-type natriuretic peptide in the emergency diagnosis of HF. *N Engl J Med,* 2002, vol. 347, 161-7
- **BAYES-GENIS A et al.** N-terminal probrain natriuretic peptide (NT-proBNP) in the emergency diagnosis and in-hospital monitoring of patients with dyspnoea and ventricular dysfunction. *Eur J Heart Fail,* 2004, vol. 6, 313-8
- **WU ALAN H.B et al.** Biological variation of the natriuretic peptides and their role in monitoring patients with heart failure. *Eur J Heart Fail,* 2004, vol. 6, 355-8
- **CHAUDHRY SI et al.** Telemonitoring in patients with heart failure. *N Engl J Med.,* 2010, vol. 363, 2301-2309
- **KOEHLER F et al.** Impact of remote telemedical management on mortality and hospitalizations in ambulatory patients with chronic heart failure: the telemedical interventional monitoring in heart failure study. *Circulation,* 2011, vol. 123, 1873-1880
- **HEIST EK et al.** Analysis of Different Device-Based Intrathoracic Impedance Vectors for Detection of Heart Failure Events (from the Detect Fluid Early from Intrathoracic Impedance Monitoring Study). *Am J Cardiol,* 2014, vol. 114, 1249-1256
- **YU CM et al.** Intrathoracic impedance monitoring in patients with heart failure: correlation with fluid status and feasibility of early warning preceding hospitalization. *Circulation,* 2005, vol. 112, 841-848
- **VOLLMANN D et al.** Clinical utility of intrathoracic impedance monitoring to alert patients with an implanted device of deteriorating chronic heart failure. *Eur Heart J,* 2007, vol. 28, 1835-1840
- **ABRAHAM WT et al.** On behalf of the FAST investigators. Superior performance of intrathoracic impedance-derived fluid index versus daily weight monitoring in heart failure patients: results of the Fluid Accumulation Status Trial (FAST). *J Card Fail,* 2009, vol. 15, 813

- **VAN VELDHUISEN DJ et al.** Intrathoracic Impedance Monitoring, Audible Patient Alerts, and Outcome in Patients With Heart Failure. *Circulation,* 2011, vol. 124, 1719-1726
- **UDELSON JE et al.** T.M.I. (Too Much Information)?. *Circulation,* 2011, vol. 124, 1697-1699
- **BRADLEY EH et al.** Hospital Strategies Associated With 30-Day Readmission Rates for Patients With Heart Failure. *Circ Cardiovasc Qual Outcomes.,* 2013, vol. 6, 444-450